# EUROPEAN PATENT APPLICATION

(11) **EP 1 288 293 A1**
(43) Date of publication of application: **05.03.2003**
(21) Application number: 01307350.7
(22) Date of filing: 30.08.2001
(51) Int. Cl.: C12N 5/08, C12N 5/06

(54) **Human neural stem cells originated from human amniotic mesenchymal cell layer**

(71) Applicant: Sakuragawa, Norio, Kodaira-shi, Tokyo 187-0032 (JP); Uchida, Saiko, Kodaira-shi, Tokyo 187-0032 (JP); SRL, INC., Tachikawa-shi, Tokyo 190-8567 (JP)
(72) Inventor: Sakuragawa, Norio, Kodaira-shi, Tokyo 187-0032 (JP); Uchida, Saiko, Kodaira-shi, Tokyo 187-0032 (JP); SRL, INC., Tachikawa-shi, Tokyo 190-8567 (JP)
(74) Representative: Walton, Seán Malcolm

(57) **Abstract**

Neural stem cells which can be provided stably and which are free from the problem of compatibility in transplantation are disclosed. The neural stem cells are separated from human amniotic mesenchymal cell layer and express nestin and musashi-1 which are markers of neural stem cells.

## Description

The present invention relates to novel neural stem cells separated from human amniotic membrane. The cells according to the present invention are useful as sources of the substances produced by nerve cells. Further, the cells according to the present invention are useful as drug delivery systems of the substances produced by nerve cells by transplanting the cells in the brain of a patient suffering from an intractable nervous disease such as Parkinson's disease or metabolic nervous diseases.

Multifunctional stem cells are undifferentiated cells which can differentiate into cells constituting various tissues, which are important in the fields of organ reconstruction and tissue engineering. As the stem cells, myeloid stem cells obtained from bone marrow and cord blood stem cells are known. However, these stem cells have problems in that they are not supplied stably. It was reported this year that a large amount of multifunctional stem cells may be recovered from human placenta. However, since placenta is originated from mother, when transplanting the cells differentiated from the stem cells originated from placenta, the compatibility of the cells must be checked in order to prevent rejection, and the cells cannot be transplanted to the patient who is not compatible with the cells, which is problematic.

An object of the present invention is to provide neural stem cells which can be supplied stably and which is free from the problem about the compatibility in transplantation.

The present inventors intensively studied to discover that neural stem cells exist in the mesenchymal cell layer of human amnion, thereby completing the present invention.

That is, the present invention provides cells separated from human amniotic mesenchymal cell layer, which express nestin and musashi-1 that are markers of neural stem cells.

By the present invention, neural stem cells which can be supplied stably and which are free from the problem about the compatibility in transplantation were first provided. Since the cells according to the present invention may be collected in a large amount together with placenta, collection of the cells is free from the ethical problem and the cells may be supplied stably. Further, since the cells according to the present invention have immunological tolerance, there is no problem about the compatibility when the cells are transplanted to a patient. Therefore, by transplanting the cells according to the present invention in the brain of a patient suffering from an intractable nervous disease such as Parkinson's disease and metabolic nervous disease, they are effective as a drug delivery system of the substances produced by nerve cells.
The present invention provides isolated neural stem cells.

As mentioned above, the cells according to the present invention are separated from human amniotic mesenchymal cell layer. The mesenchymal cell layer is located between the chorionic membrane layer and amniotic epithelial cell layer. Although amniotic membrane is a tissue originated from the fetus, it can be recovered in the state of being attached to placenta originated from mother. Further, it is a large tissue which covers the entire inner wall of uterus. Therefore, they can be obtained in a large amount. Further, since placenta and amnion attached thereto are discarded as medical wastes, there is no ethical problem in the collection of amnion.

The cells according to the present invention may be separated by peeling the amniotic epithelial cell layer + mesenchymal cell layer of human amnion from chorionic membrane layer, treating the resultant with trypsin to remove amniotic epithelial cells, and by treating the resultant with a protease. Preferred examples of the treatment with the protease include treatments with papain, with collagenase, or with a mixture of neutral protease + DNase (see Example below), but not restricted thereto.

It is confirmed by immunohistostaining that the cells according to the present invention express nestin and musashi-1. Nestin and musashi-1 are markers of neural stem cells, and it is recognized in the art that the cells expressing these markers are neural stem cells having multifunctionality (Ana Villa et al., Experimental Neurology 161, 67-84(2000)). Therefore, the cells according to the present invention are neural stem cells having multifunctionality. By culturing the cells according to the present invention in a culture medium containing B-27 (Brewer, G.J. et al., (1993) J. Neuroscience Res. 35, 567) which is an additive for culturing hippocampus cells, nestin and musashi-1 become negative, so that differentiation to nerve cells is observed. B-27 is an additive for culturing hippocampus cells, consisting essentially of biotin, L-carnitin, corticosterone, ethanolamine, D(+)-galactose, glutathione (reduced), linolenic acid, progesterone, putrescine, retinyl acetate, selenium, T3 (triodo-1-thyronein), DL-α-tocopherol, DL-α-tocopherol acetate, bovine albumin, catalase, insulin, superoxide dismutase and transferrin, and is commercially available from Invitrogen, U.S. By culturing the cells according to the present invention by suspension culture in a culture medium containing a mitogen such as fibroblast growth factor (FGF) or epidermal growth factor (EGF), cell spheres are formed. By recovering a part of the sphere and suspension-culturing the recovered cells, spheres are formed again (secondary sphere). Thus, the cells according to the present invention may be cultured in the undifferentiated state and are self-replicable.

The cultured cells obtained by primary culture or by subsequent passage, which express nestin and musashi-1 are also within the scope of the present invention.

The cells according to the present invention are originated from human amnion and the amnion is originated from the fetus, so that the cells are immunologically tolerant. That is, by immunoh stostaining, the cells according to the present invention are HLA Class I positive and HLA Class II negative. Further, Fas ligand-positive cells exist. Recently, it is thought that the reason why the amniotic tissue hardly induces rejection is that HLA Class 1b (HLA-G) is expressed and Fas ligand-positive cell exist (Ophthalmology, 42:257-269, 2000). Thus, the cells according to the present invention may be transplanted without the problem of HLA compatibility.

As will be concretely described in the Example below, the cells according to the present invention form spheres by suspension culture, and spheres (secondary spheres) are again formed by recovering a part of the primary sphere and suspension-culturing the recovered cells. Therefore, the cells according to the present invention may easily be isolated by, for example, forming secondary spheres by suspension-culturing the nestin-positive and musashi-1-positive cells selected from the cells separated by the above-mentioned treatment with protease.

The cells according to the present invention differentiate to nerve cells by being cultured in the presence of an additive for culturing hippocampus cells, such as B-27 mentioned above, and the differentiated nerve cells may be used as a source for various substances such as dopamine and choline acetyltransferase, which are produced by nerve cells. Dopamine is a substance known to drastically decrease in patients suffering from Parkinson's disease, and choline acetyltransferase is a substance known to drastically decrease in patients suffering from Alzheimer's disease. Further, since the cells are immunologically naive according to the present invention, they may be used as a drug delivery system (DDS) for delivering dopamine, acethlcholine or the like produced by transplanting the cells to the domain damaged in the Parkinson disease, dementia or the like (such as basal ganglia or striatum in case of Parkinson's disease and hippocampus in case of Alzheimer's disease). Thus, they may be used for therapy of dementia, Parkinson's disease, metabolic nervous diseases and the like. Further, a desired foreign gene may be introduced into the cells according to the present invention by a known method (such as described in Examples 1-3 of U.S. Patent No. 6,117,676), and the obtained cells may be used as a DDS for the substance encoded by the foreign gene.

The present invention will now be described by way of examples thereof. It should be noted that the Examples are presented for the illustration purpose only and should not be interpreted in any restrictive way.

### Example 1, Comparative Example 1

### 1. Separation and Culture of Cells

After obtaining informed consent of a patient, from human placenta, the amniotic epithelial cell layer + mesenchymal cell layer were obtained by separating the layers from the chorionic membrane layer. The separated layers were treated with 0.125 wt% trypsin solution at 37°C for 15 minutes. After repeating this treatment 4 times, the cells were collected by centrifuging the trypsin solution, and the cells were washed 3 times with phosphate buffer (PBS) (trypsin-treated fraction (Comparative Example 1)). The tissue block which was not digested was washed with PBS and then treated under shaking with a mixed enzyme (0.01 wt% papain, 1 mg/ml collagenase, 0.01 wt% DNase, 0.1 wt% neutral protease) at 37°C for 1 hour. The resultant was centrifuged at 1000 rpm for 10 minutes and the obtained precipitate was suspended in PBS. After passing the resultant through a 20 µm filter, the filtrate was washed 3 times with PBS (mixed enzyme-treated fraction (Example 1)).

Each fraction was primary cultured in DMEM:F-12 (1:1) medium containing 10 wt% fetal bovine serum (FBS) on a culture dish coated with collagen in an incubator containing 5% CO₂ at 37°C. The DMEM:F-12 (1:1) medium used here was 1:1 mixture of Dulbecco's modified Eagle medium (DMEM) and Ham's F-12 nutrient mixture (F-12), and is commercially available from Sigma, U.S., the mixture being generally used as a serum-free medium for culturing mammalian cells. Three days later, the cells reached confluency and the cells were treated with 0.25 wt% trypsin + 2.6 mM EDTA. The cells were then secondary cultured in the culture medium mentioned above on a 24-well collagen-coated dish. An aliquot of the culture was separated and the culture medium was changed to DMEM:F-12 (1:1) medium containing B-27 (50-fold diluted (final concentration) B-27 Supplement (50x) commercially available from Invitrogen). Three to five days later, immunohistostaining was performed by the method described below.

The primary cultured cells were treated with 0.25 wt% trypsin + 2.6 mM EDTA and the resultant was suspension-cultured in DMEM:F-12 (1:1) medium containing N2 supplement commercially available from Invitrogen (progesterone 0.63 µg/ml, putrescine 1611 µg/ml, selenite 0.52 µg/ml, insulin 500 µg/ml, human transferrin 10,000 µg/ml), 20 µg/ml of bovine FGF and 20 µg/ml of EGF (all concentrations are expressed in terms of final concentration). Two to three days later, spheres having diameters of 50 to 200 µm were formed. The spheres were sampled on a cover glass by using a cell-collection centrifuge, and immunostaining was performed by the method described below. After treating the spheres with 0.25 wt% trypsin + 2.6 mM EDTA, the resulting cells were again suspension-cultured in the medium described above.

### 2. Immunostaining

Immunohistostaining was carried out by a conventional method using anti-human nestin polyclonal antibody or anti-human musashi-1 monoclonal antibody as a primary antibody, and using an anti-rabbit IgG-rhodamine (1:100, commercially available from Chemicon) or anti-rabbit IgG-FITC (commercially available from ZYMED) as a secondary antibody. More concretely, the immunohistostaining was carried out as follows: The cultured cells or amnion tissue were fixed with 4 wt% paraformaldehyde for 1 minute and the resultant was incubated with the above-mentioned primary antibody at room temperature for 2 hours. The resultant was then incubated with the secondary antibody diluted with 0.3 wt% Triton X100 (trademark) at room temperature for 2 hours. The immunoblotted cells were observed with a fluorescence microscope (IX 10, commercially available from Olympus), and the confocal image obtained by using a laser scanning microscope (Fluoview, commercially available from Olympus). Further, using commercially available monoclonal antibodies to other cell markers, immunohistostaining was carried out for CK19 (SANTA CRUZ), vimentin (PROGEN), Gal C (SIGMA) and β-tub-III (SIGMA) (the mentioned manufacturers are those commercializing monoclonal antibodies to the mentioned cell markers) in the same manner as mentioned above. Further, anti-Fas ligand antibody (SANTA CRUZ), anti-HLA Class I antibody (HLA-A, B, C; ANSEL) or anti-HLA Class II (HLA-DP, DQ, DR; ANSEL) was used as the primary antibody.

### 3. Results

The cells according to the present invention (Example 1) obtained from the mixed enzyme-treated fraction, which were cultured in DMEM:F-12 (1:1) containing 10% FBS for 3 days on the collagen-coated culture dish, were CK19-/vimentin++/nestin+/musashi-1+/Gal C+/β-tub-III+ by immunostaining. As mentioned above, it is recognized in the art that the cells expressing nestin and musashi-1 are neural stem cells. Therefore, it was proved that the cells according to the present invention are neural stem cells. The above-described culture was also carried out in the presence of 5-bromo-2'-deoxy-uridine (5BrDU) (ROCHE DIAGNOSTICS), and 5BrDU in the cells was detected with a commercially available kit (ROCHE DIAGNOSTICS). The result was weakly positive, so that the cells were in the stage of mitosis. By culturing the cells in a B-27-containing culture medium, they were changed to vimentin±/nestin-/musashi-1-/Gal C±/β-tub-III++. Thus, the neural stem cell markers disappeared, so that differentiation into nerve cells was suggested.

By suspension-culturing the cells according to the present invention in N2-, bovine FGF- and EGF-containing medium, spheres with diameters of 50 to 200 µm were formed 2 to 3 days after the beginning of the suspension culture. By culturing an aliquot of the spheres in the same manner, secondary spheres were formed similarly. Thus, it was proved that the cells according to the present invention are self-replicable, and can be cultured in undifferentiated state in the presence of a mitogen such as FGF or EGF.

On the other hand, the cells (Comparative Example 1) obtained from the trypsin-treated fraction, which were cultured in DMEM:F-12 (1:1) containing 10% FBS for 3 days on the collagen-coated culture dish, were CK19+/vimentin+/nestin/musashi-1-. Further, no spheres were formed by the above-mentioned suspension culture. Thus, it was proved that neural stem cells are not included in the amniotic epithelial cells.

## Claims

1. Cells separated from human amniotic mesenchymal cell layer, which express nestin and musahi-1 that are markers of neural stem cells.

2. A method of obtaining cells according to claim 1, the method comprising:
peeling the amniotic epithelial layer and mesenchymal cell layer of human amnion from the chorionic membrane layer;
treating the resultant material with trypsin to remove amniotic epithelial cells; and
treating the resultant material with a protease.

3. A method according to claim 2 further comprising immunohistostaining resultant cells to detect expression of nestin and musashi-1.

4. A method according to claim 2 or claim 3 further comprising culturing the cells.
